# EUROPEAN PATENT APPLICATION

(11) **EP 4 721 721 A1**
(43) Date of publication of application: **08.04.2026**
(21) Application number: 24315447.3
(22) Date of filing: 01.10.2024
(51) Int. Cl.: A61K 6/62, A61K 6/887, B33Y 70/00, B33Y 80/00, C08F 222/10, C08F 290/06

(54) **CURABLE COMPOSITION COMPRISING AN ALKOXYLATED ISOSORBIDE DI(METH)ACRYLATE**

(71) Applicant: ARKEMA FRANCE, 92800 Puteaux (FR)
(72) Inventor: FEILLEE, Noémi, 60550 Verneuil-en-Halatte (FR); KELKAR, Sneha, Exton, PA 19341 (US); EVRARD, Didier, 60550 Verneuil-en-Halatte (FR); BRAZ RIBEIRO, Frederico, 60550 Verneuil-en-Halatte (FR)
(74) Representative: Renard, Emmanuelle

(57) **Abstract**

The present invention relates to a curable composition comprising: a) at least one alkoxylated isosorbide di(meth)acrylate, b) at least one (meth)acrylate-functionalized oligomer, and c) at least one (meth)acrylate-functionalized monomer. The invention also relates to a process for the preparation of a cured product, especially a dental material, comprising curing this curable composition, preferably by exposing the curable composition to radiation such as UV, near-UV, visible, infrared, near-infrared and/or electron beam radiation.

## Description

### TECHNICAL FIELD

The present invention relates to a curable composition comprising: a) at least one alkoxylated isosorbide di(meth)acrylate, b) at least one (meth)acrylate-functionalized oligomer, and c) at least one (meth)acrylate-functionalized monomer. The invention also relates to a process for the preparation of a cured product, especially a dental material, comprising curing this curable composition, preferably by exposing the curable composition to radiation such as UV, near-UV, visible, infrared, near-infrared and/or electron beam radiation.

### TECHNICAL BACKGROUND

Radiation curable compositions containing ethylenically unsaturated compounds such as (meth)acrylate monomers can be polymerized by exposure to radiation, such as ultraviolet (UV) light, to produce a number of materials. Among these materials, mention can be made of dental materials such as dental models, try-ins, denture teeth, denture base, crown and bridge appliances and orthodontic appliances. These appliances need to have specific mechanical properties such as high stress at break, high tensile modulus, high flexural modulus and high flexural strength, so as to withstand deformations when in use. In addition, since they are typically produced by additive processes such as stereolithography, these materials need to be obtainable from low viscosity resin compositions.

In order to satisfy these needs, it has been suggested to blend polyfunctional (meth)acrylate monomers and optionally monofunctional (meth)acrylate monomers with an aliphatic urethane (meth)acrylate oligomer. The polyfunctional (meth)acrylate monomers may comprise bisphenol A dimethacrylate with 3 ethoxy groups (SR348C^{®} from Arkema) or bis-GMA, which is the addition product of methacrylic acid with bisphenol A (BPA) diglycidyl ether (US2020/199346). Although these compounds confer beneficial properties to the material, they are known to contain residual BPA, which has proven to be an endocrine disruptor for humans and environmental organisms. Therefore, it would be desirable to substitute these compounds with monomers acting as reactive diluents so as to provide monomer compositions with lower toxicity and similar viscosity and sensitivity to curing than compositions based on (meth)acrylated BPA. It would also be desirable to provide materials having mechanical properties comparable to those of (meth)acrylated BPA-based materials. It would further be desirable to replace BPA-based compounds with alternative reactants which are more friendly to the environment.

In this context, it has already been suggested to substitute an isosorbide derivative for these BPA derivatives, in the manufacture of materials intended for use as dental cements (KR101916876) and dental adhesives (JP7104510) which require high flexural strength. Various dental cement compositions have thus been proposed, comprising an alkoxylated isosorbide di(meth)acrylate (see also WO2011/048739, JP6892319). It has also been suggested to use this isosorbide derivative in the manufacture of other curable compositions by stereolithography, for instance of sealants for electronic devices (KR-10-2022-0096594).

However, to the best of the inventors' knowledge, it has never been suggested to use this isosorbide derivative in combination with both a (meth)acrylate oligomer and a (meth)acrylate oligomer, let alone in the manufacture of items such as dental materials by 3D printing.

### SUMMARY OF THE INVENTION

This invention pertains to a curable composition comprising:
a) at least one alkoxylated isosorbide di(meth)acrylate,
b) at least one (meth)acrylate-functionalized oligomer, and
c) at least one (meth)acrylate-functionalized monomer.

It is also directed to a process for the preparation of a cured product, comprising curing this curable composition, preferably by exposing the curable composition to radiation such as UV, near-UV, visible, infrared, near-infrared and/or electron beam radiation.

### DETAILED DESCRIPTION

### Definitions

The wording "polymerizable compounds" refers to compounds (monomers and/or oligomers) having a polymerizable carbon-carbon double bond. A polymerizable carbon-carbon double bond is a carbon-carbon double bond that can react with another carbon-carbon double bond in a polymerization reaction. A polymerizable carbon-carbon double bond is generally comprised in a group selected from acrylate, methacrylate, cyanoacrylate, acrylamide, methacrylamide, styrene, maleate, fumarate, itaconate, allyl, propenyl, vinyl and combinations thereof, preferably selected from acrylate, methacrylate, cyanoacrylate, allyl and vinyl, more preferably selected from acrylate and methacrylate. The carbon-carbon double bonds of a phenyl ring are not considered as polymerizable carbon-carbon double bonds.

### Curable composition

The curable (or polymerizable) composition of this invention comprises three key components, namely:
a) at least one alkoxylated isosorbide di(meth)acrylate,
b) at least one (meth)acrylate-functionalized oligomer, and
c) at least one (meth)acrylate-functionalized monomer.

### Alkoxylated isosorbide di(meth)acrylate

The curable composition of the invention comprises at least one alkoxylated isosorbide di(meth)acrylate, also referred to as component a).

In an embodiment, component a) comprises or consists of at least one alkoxylated isosorbide di(meth)acrylate having from 1 to 5 oxyalkylene moieties selected from oxyethylene, oxypropylene, oxybutylene and mixtures thereof, preferably at least one alkoxylated isosorbide di(meth)acrylate having from 1 to 5 oxypropylene moieties; more preferably at least one alkoxylated isosorbide dimethacrylate with 1 to 5 oxypropylene moieties.

In a preferred embodiment, component a) comprises or consists of at least one alkoxylated isosorbide di(meth)acrylate according to the following formula (1): 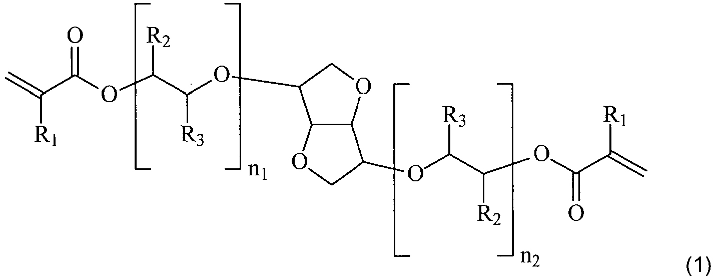 wherein
n₁ and n₂ are independently from 0 to 5 and the sum of n₁ + n₂ ranges from 1 to 5,
each R₁ is independently H or CH₃, preferably CH₃, and
R₂ and R₃ are independently selected from H or CH₃, preferably in each -OCHR₂-CHR₃-unit one of R₂ or R₃ is H and the other is CH₃.

Component a) may represent from 5 to 70 wt.%, preferably from 10 to 60 wt.%, more preferably from 20 to 50 wt.% of the total weight of polymerizable compounds in the composition.

An alkoxylated isosorbide di(meth)acrylate may be prepared by (meth)acrylating an isosorbide alkylene oxide adduct. Said adduct may be prepared by alkoxylating isosorbide. Isosorbide can be produced by various methods, for instance by dehydrating sorbitol by the action of various dehydration catalysts, particularly strong acid catalysts, either in water or in an organic solvent, then purification according to any known method for purifying anhydrous sugar alcohols.

For producing an alkoxylated isosorbide di(meth)acrylate as used in this invention, the following steps may independently be carried out:
(1) esterification of an isosorbide alkylene oxide adduct with a (meth)acrylic acid halide or a (meth)acrylic anhydride;
(2) transesterification of an isosorbide alkylene oxide adduct with a (meth)acrylic acid ester of a lower alcohol such as methyl methacrylate; or
(3) dehydration condensation of an isosorbide alkylene oxide adduct with (meth)acrylic acid in the presence of carbodiimide-based dehydration condensation agents such as dicyclohexylcarbodiimide and water-soluble carbodiimide; or
(4) dehydration condensation of an isosorbide alkylene oxide adduct with (meth)acrylic acid in the presence of an acid catalyst.

### (Meth)acrylate-functionalized oligomer

The curable composition of the invention comprises at least one (meth)acrylate-functionalized oligomer, also referred to as component b).

The term "(meth)acrylate-functionalized oligomer" means an oligomer comprising at least one (meth)acrylate group, in particular an acrylate group. The term "(meth)acrylate group" encompasses acrylate groups (-O-CO-CH=CH₂) and methacrylate groups (-O-CO-C(CH₃)=CH₂).

Component b) is distinct from components a) and c).

In a preferred embodiment, component b) comprises or consists of at least one (meth)acrylate-functionalized oligomer having a number average molecular weight equal or more than 600 g/mol, in particular 800 to 15,000 g/mol, more particularly 1,000 to 5,000 g/mol.

Moreover, component b) preferably comprises or consists of at least one (meth)acrylate-functionalized oligomer having 1 to 10 (meth)acrylate groups, in particular 2 to 6 (meth)acrylate groups, more particularly 2 (meth)acrylate groups.

In particular, component b) may comprise or consist of one or more (meth)acrylate-functionalized oligomers selected from (meth)acrylate-functionalized urethane oligomers (sometimes also referred to as "urethane (meth)acrylate oligomers," "polyurethane (meth)acrylate oligomers" or "carbamate (meth)acrylate oligomers"), (meth)acrylate-functionalized epoxy oligomers (sometimes also referred to as "epoxy (meth)acrylate oligomers"), (meth)acrylate-functionalized polydiene oligomers (sometimes also referred to as "polydiene (meth)acrylate oligomers"), (meth)acrylate-functionalized polycarbonate oligomers (sometimes also referred to as "polycarbonate (meth)acrylate oligomers"), (meth)acrylate-functionalized polyester oligomers (sometimes also referred to as "polyester (meth)acrylate oligomers"), (meth)acrylate-functionalized (meth)acrylic oligomers (sometimes also referred to as "(meth)acrylic (meth)acrylate oligomers") and mixtures thereof.

Preferably, component b) comprises or consists of a urethane (meth)acrylate oligomer.

Suitable urethane (meth)acrylate oligomers include, for example, aliphatic polyester-based urethane di- and tetra-acrylate oligomers, aliphatic polyether-based urethane di- and tetra-acrylate oligomers, as well as aliphatic polyester/polyether-based urethane di- and tetra-acrylate oligomers.

Urethane (meth)acrylate oligomers may contain two, three, four or more (meth)acrylate functional groups per molecule.

Advantageously, the urethane (meth)acrylate oligomer comprises an aliphatic urethane (meth)acrylate oligomer having two (meth)acrylate groups.

Urethane (meth)acrylate oligomers may be prepared by reacting a polyisocyanate (e.g., an aliphatic, cycloaliphatic and/or aromatic diisocyanate or triisocyanate) with a polyol (for example a polyester polyol, a polyether polyol, a polycarbonate polyol, a polycaprolactone polyol, a polyorganosiloxane polyol such as a polydimethylsiloxane polyol, or a polydiene polyol such as a polybutadiene polyol, or combinations thereof) and a hydroxyl-functionalized (meth)acrylate (such as 2-hydroxyethyl (meth)acrylate or 2- or 3-hydroxypropyl (meth)acrylate). Any order of addition may be practiced to prepare the urethane (meth)acrylate, as is known in the art. For example, a hydroxyl-functionalized (meth)acrylate may first be reacted with a polyisocyanate to obtain an isocyanate-functionalized (meth)acrylate, which may then be reacted with a polyol. In yet another embodiment, a polyisocyanate may first be reacted with a polyol to obtain an isocyanate-functionalized prepolymer, which is thereafter reacted with a hydroxyl-functionalized (meth)acrylate. Alternatively, all the components may be combined and reacted at the same time.

Exemplary polyester (meth)acrylate oligomers include the reaction products of a (meth)acrylating agent (such as (meth)acrylic acid, (meth)acrylic anhydride, (meth)acryloyl halide, mixtures thereof or synthetic equivalents thereof) with a polyester polyol. The reaction process may be conducted such that all or essentially all of the hydroxyl groups of the polyester polyol have been (meth)acrylated, particularly in cases where the polyester polyol is difunctional. A polyester polyol can be made by polycondensation reactions of a polyol (in particular, a diol) and a polycarboxylic acid functional compound (in particular, a dicarboxylic acid or a cyclic anhydride). The polyol and polycarboxylic acid functional compounds can each have linear, branched, cycloaliphatic or aromatic structures and can be used individually or as mixtures.

Examples of suitable epoxy (meth)acrylates oligomers include the reaction products of a (meth)acrylating agent as defined above with an epoxy resin (such as a polyglycidyl ether or ester). The epoxy resin may, in particular, be selected from 3,4-epoxycyclohexylmethyl-3',4'-epoxycyclohexanecarboxylate, 2-(3,4-epoxycyclohexyl-5,5-spiro-3,4-epoxy) cyclohexane-1,4-dioxane, bis(3,4-epoxycyclohexylmethyl)adipate, bis(3,4-epoxy-6-methylcyclohexylmethyl)adipate, 3,4-epoxy-6-methylcyclohexyl-3',4'-epoxy-6'-methylcyclohexanecarboxylate, dicyclopentadiene diepoxide, a bisphenol diglycidyl ether, 1,4-butanediol diglycidyl ether, 1,6-hexanediol diglycidyl ether, glycerol triglycidyl ether, trimethylolpropane triglycidyl ether, polyethylene glycol diglycidyl ether, polypropylene glycol diglycidyl ether, polyglycidyl ethers of a polyether polyol obtained by the addition of one or more alkylene oxides to an aliphatic polyhydric alcohol such as ethylene glycol, propylene glycol and glycerol, diglycidyl esters of aliphatic C6-C22 dibasic acids, glycidyl esters of C30-36 dimers of fatty acids, an epoxidized vegetable oil (such as epoxidized soybean oil and epoxidized linseed oil), epoxidized polybutadiene, and the like.

Suitable (meth)acrylic (meth)acrylate oligomers include oligomers which may be described as substances having an oligomeric (meth)acrylic backbone which is functionalized with one or (meth)acrylate groups (which may be at a terminus of the oligomer or pendant to the acrylic backbone). The (meth)acrylic backbone may be a homopolymer, random copolymer or block copolymer comprised of repeating units of (meth)acrylic monomers. The (meth)acrylic monomers may be any monomeric (meth)acrylate such as C1-C6 alkyl (meth)acrylates as well as functionalized (meth)acrylates such as (meth)acrylates bearing hydroxyl, carboxylic acid and/or epoxy groups. (Meth)acrylic (meth)acrylate oligomers may be prepared using any procedures known in the art, such as by oligomerizing monomers, at least a portion of which are functionalized with hydroxyl, carboxylic acid and/or epoxy groups (e.g., hydroxyalkyl(meth)acrylates, (meth)acrylic acid, glycidyl (meth)acrylate) to obtain a functionalized oligomer intermediate, which is then reacted with one or more (meth)acrylate-containing reactants to introduce the desired (meth)acrylate functional groups.

Component b) may represent from 5 to 60 wt.%, preferably from 10 to 55 wt%., more preferably from 10 to 50 wt.% of the total weight of polymerizable compounds in the composition.

In a first embodiment, component b) may represent from 30 to 60 wt.%, preferably from 35 to 55 wt%, more preferably from 40 to 50 wt.% of the total weight of polymerizable compounds in the composition.

In a second embodiment, component b) may represent from 5 to 30 wt.%, preferably from 7 to 25 wt%, more preferably from 10 to 20 wt.% of the total weight of polymerizable compounds in the composition.

### (Meth)acrylate-functionalized monomer

The curable composition of the invention comprises at least one (meth)acrylate-functionalized monomer, also referred to as component c).

As used herein, the term "(meth)acrylate-functionalized monomer" means a monomer comprising a (meth)acrylate group. The term "(meth)acrylate group" encompasses acrylate groups (-O-CO-CH=CH₂) and methacrylate groups (-O-CO-C(CH₃)=CH₂).

Component c) is distinct from components a) and b).

A (meth)acrylate-functionalized monomer may have a molecular weight of less than 600 g/mol, in particular from 100 to 550 g/mol, more particularly 200 to 500 g/mol.

A (meth)acrylate-functionalized monomer may have 1 to 6 (meth)acrylate groups, in particular 1 to 3 (meth)acrylate groups.

Component c) may comprise a mixture of (meth)acrylate-functionalized monomers having different functionalities. For example component c) may comprise a mixture of a (meth)acrylate-functionalized monomer containing a single acrylate or methacrylate group per molecule (referred to herein as "mono(meth)acrylate-functionalized monomers") and a (meth)acrylate-functionalized monomer containing 2 or more, preferably 2 to 6, acrylate and/or methacrylate groups per molecule (referred to herein as "poly(meth)acrylate-functionalized monomer"),

Component c) may comprise or consist of a mono(meth)acrylate-functionalized monomer.

Examples of suitable mono(meth)acrylate-functionalized monomers include, but are not limited to, mono-(meth)acrylate esters of aliphatic alcohols (wherein the aliphatic alcohol may be straight chain, branched or alicyclic and may be a mono-alcohol, a di-alcohol or a polyalcohol, provided only one hydroxyl group is esterified with (meth)acrylic acid); mono-(meth)acrylate esters of aromatic alcohols (such as phenols, including alkylated phenols); mono-(meth)acrylate esters of alkylaryl alcohols (such as benzyl alcohol); mono-(meth)acrylate esters of oligomeric and polymeric glycols such as diethylene glycol, triethylene glycol, dipropylene glycol, tripropylene glycol, polyethylene glycol, and polypropylene glycol); mono-(meth)acrylate esters of monoalkyl ethers of glycols and oligoglycols; mono-(meth)acrylate esters of alkoxylated (e.g., ethoxylated and/or propoxylated) aliphatic alcohols (wherein the aliphatic alcohol may be straight chain, branched or alicyclic and may be a mono-alcohol, a di-alcohol or a polyalcohol, provided only one hydroxyl group of the alkoxylated aliphatic alcohol is esterified with (meth)acrylic acid); mono-(meth)acrylate esters of alkoxylated (e.g., ethoxylated and/or propoxylated) aromatic alcohols (such as alkoxylated phenols); caprolactone mono(meth)acrylates; and mixtures thereof.

The following compounds are specific examples of mono(meth)acrylate-functionalized monomers suitable for use in component c): methyl (meth)acrylate; ethyl (meth)acrylate; n-propyl (meth)acrylate; n-butyl (meth)acrylate; isobutyl (meth)acrylate; n-hexyl (meth)acrylate; 2-ethylhexyl (meth)acrylate; n-octyl (meth)acrylate; isooctyl (meth)acrylate; n-decyl (meth)acrylate; n-dodecyl (meth)acrylate; tridecyl (meth)acrylate; tetradecyl (meth)acrylate; hexadecyl (meth)acrylate; 2-hydroxyethyl (meth)acrylate; 2- and 3-hydroxypropyl (meth)acrylate; 2-methoxyethyl (meth)acrylate; 2-ethoxyethyl (meth)acrylate; 2- and 3-ethoxypropyl (meth)acrylate; tetrahydrofurfuryl (meth)acrylate; 2-(2-ethoxyethoxy)ethyl (meth)acrylate; cyclohexyl (meth)acrylate; glycidyl (meth)acrylate; isodecyl (meth)acrylate; lauryl (meth)acrylate; 2-phenoxyethyl (meth)acrylate; phenol (meth)acrylates; nonylphenol (meth)acrylates; benzyl (meth)acrylate, cyclic trimethylolpropane formal (meth)acrylate; (2,2-dimethyl-1,3-dioxolan-4-yl)methyl (meth)acrylate; (2-ethyl-2-methyl-1,3-dioxolan-4-yl)methyl (meth)acrylate; glycerol formal methacrylate; isobornyl (meth)acrylate; tricyclodecanemethanol (meth)acrylate; tert-butylcyclohexyl (meth)acrylate; trimethylcyclohexyl (meth)acrylate; diethylene glycol monomethyl ether (meth)acrylate; diethylene glycol monoethyl ether (meth)acrylate; diethylene glycol monobutyl ether (meth)acrylate; triethylene glycol monoethyl ether (meth)acrylate; ethoxylated lauryl (meth)acrylate; methoxy polyethylene glycol (meth)acrylates; hydroxyl ethyl-butyl urethane (meth)acrylates; 3-(2-hydroxyalkyl)oxazolidinone (meth)acrylates; a caprolactone mono(meth)acrylate; as well as the alkoxylated (i.e. ethoxylated and/or propoxylated) derivatives thereof; and combinations thereof.

Component c) may comprise or consist of a poly(meth)acrylate-functionalized monomer.

Examples of suitable poly(meth)acrylate-functionalized monomers include acrylate and methacrylate esters of polyols selected from ethylene glycol, di-, tri- or tetraethylene glycol, 1,2- or 1,3-propylene glycol, di-, tri- or tetra(1,2-propylene glycol), di-, tri- or tetra(1,3-propylene glycol), 1,2-, 1,3- or 1,4-butylene glycol, di-, tri- or tetra(1,4-butylene glycol), 1,5-pentanediol, 1,6-hexanediol, 1,7-heptanediol, 1,8-octanediol, 1,9-nonanediol, 1,10-decanediol, 1,12-dodecanediol, 2-methyl-1,3-propanediol, 2,2-dimethyl-1,3-propanediol, 2,2-diethyl-1,3-propanediol, 2-methyl-2-ethyl-1,3-propanediol, 3-methyl-1,5-pentanediol, 3,3-dimethyl-1,5-pentanediol, 2,4-diethyl-1,5-pentanediol, 3-butyl-3-ethyl-1,5-pentane diol, 2,2,4-trimethyl 1,5-pentanediol, cyclohexanediol, cyclohexane-1,4-dimethanol, norbornene dimethanol, norbornane dimethanol, tricyclodecanediol, tricyclodecane dimethanol, hydrogenated bisphenol A, B, F or S, a dianhydrohexitol (i.e. isosorbide, isomannide, isoidide), as well as the alkoxylated (i.e. ethoxylated and/or propoxylated) derivatives thereof; and mixtures thereof. Such polyols may be fully or partially esterified (with (meth)acrylic acid, (meth)acrylic anhydride, (meth)acryloyl chloride or the like), provided they contain at least two (meth)acrylate functional groups per molecule.

Exemplary poly(meth)acrylate-functionalized monomers may include bisphenol A di(meth)acrylate; hydrogenated bisphenol A di(meth)acrylate; ethylene glycol di(meth)acrylate; diethylene glycol di(meth)acrylate; triethylene glycol di(meth)acrylate; tetraethylene glycol di(meth)acrylate; polyethylene glycol di(meth)acrylate; propylene glycol di(meth)acrylate; dipropylene glycol di(meth)acrylate; tripropylene glycol di(meth)acrylate; tetrapropylene glycol di(meth)acrylate; polypropylene glycol di(meth)acrylate; polytetramethylene glycol di(meth)acrylate; 1,2-butanediol di(meth)acrylate; 2,3-butanediol di(meth)acrylate; 1,3-butanediol di(meth)acrylate; 1,4-butanediol di(meth)acrylate; 1,5-pentanediol di(meth)acrylate; 1,6-hexanediol di(meth)acrylate; 1,8-octanediol di(meth)acrylate; 1,9-nonanediol di(meth)acrylate; 1,10-decanediol di(meth)acrylate; 1,12-dodecanediol di(meth)acrylate; neopentyl glycol di(meth)acrylate; 2-methyl-2,4-pentanediol di(meth)acrylate; polybutadiene di(meth)acrylate; cyclohexane-1,4-dimethanol di(meth)acrylate; tricyclodecane dimethanol di(meth)acrylate; a non-alkoxylated isosorbide di(meth)acrylate; glyceryl di(meth)acrylate; glyceryl tri(meth)acrylate; trimethylolethane tri(meth)acrylate; trimethylolethane di(meth)acrylate; trimethylolpropane tri(meth)acrylate; trimethylolpropane di(meth)acrylate; pentaerythritol di(meth)acrylate; pentaerythritol tri(meth)acrylate; pentaerythritol tetra(meth)acrylate, di(trimethylolpropane) diacrylate; di(trimethylolpropane) triacrylate; di(trimethylolpropane) tetraacrylate; sorbitol penta(meth)acrylate; di(pentaerythritol) tetraacrylate; di(pentaerythritol) pentaacrylate; di(pentaerythritol) hexa(meth)acrylate; tris (2-hydroxyethyl) isocyanurate tri(meth)acrylate; as well as the alkoxylated (i.e. ethoxylated and/or propoxylated) derivatives thereof; and mixtures thereof; and mixtures thereof.

Another example of a (meth)acrylate-functionalized monomer is a urethane di(meth)acrylate monomer.

A urethane di(meth)acrylate monomer may have 2 (meth)acrylate groups and two urethane bonds. A urethane di(meth)acrylate monomer may be the reaction product of a diisocyanate and a hydroxyalkyl (meth)acrylate.

In particular, component c) may comprise a urethane di(meth)acrylate monomer according to the following formula (2): 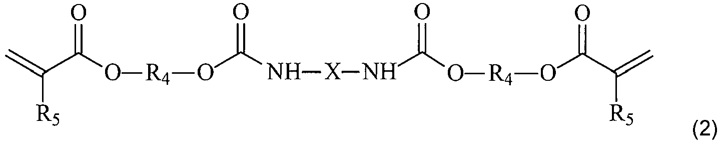 wherein
each R₄ is independently -CH₂-CH₂-, -CH₂-CH(CH₃)- or -CH(CH₃)-CH₂-, preferably -CH₂-CH₂-;
each R₅ is independently H or methyl, preferably methyl;
X is the residue of a diisocyanate, preferably the residue of isophorone diisocyanate or 2,2,4- or 2,4,4-trimethylhexamethylene diisocyanate.

In a particularly preferred embodiment, component c) comprises at least one (meth)acrylate-functionalized monomer selected from a (meth)acrylate-functionalized monomer having a cyclic moiety The cyclic moiety may be monocyclic, bicyclic or tricyclic, including bridged, fused and/or spirocyclic ring systems. The cyclic moiety may be carbocyclic (all of the ring atoms are carbons), or heterocyclic (the rings atoms consist of at least two elements). The cyclic moiety may be aliphatic, aromatic or a combination of aliphatic and aromatic. In particular, the cyclic moiety may comprise a ring or ring system selected from cycloalkyl, heterocycloalkyl, aryl, heteroaryl and combinations thereof.

More particularly, the cyclic moiety may comprise a ring or ring system selected from phenyl, cyclopentyl, cyclohexyl, norbornyl, tricyclodecanyl, dicyclopentadienyl, oxiranyl, oxetanyl, tetrahydrofuranyl, tetrahydropyranyl, dioxolanyl, dioxanyl, dioxaspirodecanyl and dioxaspiroundecanyl. The ring or ring system may be optionally substituted by one or more groups selected from hydroxyl, alkoxy, alkyl, hydroxyalkyl, cycloalkyl, aryl, alkylaryl and arylalkyl.

In a particularly preferred embodiment, component c) comprises at least one (meth)acrylate-functionalized monomer selected from cyclic trimethylolpropane formal (meth)acrylate; isobornyl (meth)acrylate; tricyclodecanemethanol (meth)acrylate; tert-butylcyclohexanol (meth)acrylate; trimethylcyclohexanol (meth)acrylate, 2-phenoxyethyl (meth)acrylate, 2-hydroxyethyl (meth)acrylate, ethoxylated cumylphenol (meth)acrylate, 2-hydroxy-3-phenoxypropyl (meth)acrylate;triethylene glycol di(meth)acrylate; tetraethylene glycol di(meth)acrylate; polyethylene glycol di(meth)acrylate; polypropylene glycol di(meth)acrylate; 1,6-hexanediol di(meth)acrylate; 1,8-octanediol di(meth)acrylate; 1,9-nonanediol di(meth)acrylate; 1,10-decanediol di(meth)acrylate; 1,12-dodecanediol di(meth)acrylate; neopentyl glycol di(meth)acrylate; 2-methyl-2,4-pentanediol di(meth)acrylate; polybutadiene di(meth)acrylate; cyclohexane-1,4-dimethanol di(meth)acrylate; tricyclodecane dimethanol di(meth)acrylate; glyceryl di(meth)acrylate; pentaerythritol tetra(meth)acrylate, tris (2-hydroxyethyl) isocyanurate tri(meth)acrylate, as well as the alkoxylated (i.e. ethoxylated and/or propoxylated) derivatives thereof; and mixtures thereof.

Component c) may represent from 5 to 70 wt.%, preferably from 10 to 60 wt.%, more preferably from 20 to 50 wt.% of the total weight of polymerizable compounds in the composition.

### Other constituents

The curable composition of the invention may further comprise one or more other constituents.

The composition of this invention typically comprises at least one radical initiator.

In addition, it may comprise one or more compounds selected from: ethylenically unsaturated compounds other than components a), b) and c); amine synergists; additives; solvents; and mixtures thereof.

### Radical initiator

The radical initiator may be selected from peroxides, photoinitiators, and mixtures thereof.

Suitable peroxides for this purpose may include any compound, in particular any organic compound, that contains at least one peroxy (-O-O-) moiety, such as, for example, dialkyl, diaryl and aryl/alkyl peroxides, hydroperoxides, percarbonates, peresters, peracids, acyl peroxides and the like.

Photoinitiators are compounds that can generate free radicals upon exposure to light of an appropriate wavelength and/or intensity. Photoinitiators can adopt two different modes of action, and are classified by mode of action as Norrish Type I and Norrish Type II photoinitiators. As used herein, the term "activity" with reference to Norrish Type I and Norrish Type II activity is intended to relate to Norrish photoinitiation and analogous reactions. For instance, a photoinitiator having Norrish Type I activity within the scope of this invention would be a photoinitiator characterized by a cleavage reaction into two radical fragments of the original photoinitiator on exposure to light. For an initiator having Norrish Type II activity, exposure to light causes the abstraction of an atom, such as hydrogen, to generate the radical. The photoinitiator may be a photoinitiator having Norrish type I activity and/or Norrish type II activity, more particularly a photoinitiator having Norrish type II activity. Non-limiting types of photoinitiators suitable for use in the composition of the invention include, for example, benzoins, benzoin ethers, acetophenones, α-hydroxy acetophenones, benzil, benzil ketals, anthraquinones, phosphine oxides, acylphosphine oxides, α-hydroxyketones, phenylglyoxylates, α-aminoketones, benzophenones, thioxanthones, xanthones, acridine derivatives, phenazine derivatives, quinoxaline derivatives, triazine compounds, benzoyl formates, aromatic oximes, metallocenes, acylsilyl or acylgermanyl compounds, camphorquinones, polymeric derivatives thereof, and mixtures thereof. Examples of suitable photoinitiators include, but are not limited to, 2-methylanthraquinone, 2-ethylanthraquinone, 2-chloroanthraquinone, 2-benzyanthraquinone, 2-t-butylanthraquinone, 1,2-benzo-9,10-anthraquinone, benzoin ethers, benzoin, benzoin methyl ether, benzoin ethyl ether, benzoin isopropyl ether, alpha-methylbenzoin, alpha-phenylbenzoin, Michler's ketone, acetophenones such as 2,2-dialkoxybenzophenones and 1-hydroxyphenyl ketones, benzophenone, 4,4'-bis-(diethylamino) benzophenone, acetophenone, 2,2-diethyloxyacetophenone, , 2-isopropylthioxanthone, thioxanthone, diethyl thioxanthone, 1,5-acenaphthylene, benzil, α-hydroxyketone, 2,4,6-trimethylbenzoyldiphenyl phosphine oxide, , 2,2-dimethoxy-1,2-diphenylethanone, 1-hydroxycyclohexyl phenyl ketone, 2-methyl-1-[4-(methylthio) phenyl]-2-morpholinopropanone, 2-hydroxy-2-methyl-1-phenyl-propanone, oligomeric α-hydroxy ketone, benzoyl phosphine oxides, phenylbis(2,4,6-trimethylbenzoyl)phosphine oxide, ethyl(2,4,6-trimethylbenzoyl)phenyl phosphinate, anisoin, anthraquinone, anthraquinone-2-sulfonic acid sodium salt monohydrate, (benzene) tricarbonylchromium, , benzoin isobutyl ether, benzophenone/1-hydroxycyclohexyl phenyl ketone 50/50 blend, 3,3',4,4'-benzophenonetetracarboxylic dianhydride, 4-benzoylbiphenyl, 2-benzyl-2-(dimethylamino)-4'-morpholinobutyrophenone, 4,4'-bis(diethylamino)benzophenone, 4,4'-bis(dimethyl-amino)benzophenone, camphorquinone, 2-chlorothioxanthen-9-one, dibenzosuberenone, 4,4'-dihydroxybenzophenone, , 4-(dimethylamino)benzophenone, 4,4'-dimethylbenzil, 2,5-dimethylbenzophenone, 3,4-dimethylbenzophenone, diphenyl(2,4,6-trimethylbenzoyl) phosphine oxide /2-hydroxy-2-methylpropiophenone 50/50 blend, 4'-ethoxyacetophenone, 2,4,6-trimethylbenzoyldiphenylphosphine oxide, 3'-hydroxyacetophenone, 4'-hydroxyacetophenone, 3-hydroxybenzophenone, 4-hydroxybenzophenone, 2-methylbenzophenone, 3-methylbenzophenone, methybenzoylformate, phenanthrenequinone, 4'-phenoxyacetophenone, (cumene)cyclopentadienyl iron(ii) hexafluorophosphate, 9,10-diethoxy and 9,10-dibutoxyanthracene, 2-ethyl-9,10-dimethoxyanthracene, and combinations thereof.

In particular, the radical initiator may comprise a photoinitiator selected from a benzophenone such as SpeedCure^{®} BP (benzophenone), SpeedCure^{®} 7005 (polymeric benzophenone), SpeedCure^{®} 7006 (polymeric benzophenone), SpeedCure^{®} EMK (4,4'-bis(diethylamino)benzophenone) or SpeedCure^{®} BMS (4-benzoyl-4'-methyldiphenyl sulphide); a thioxanthone such as SpeedCure^{®} 7010 (polymeric thioxanthone), SpeedCure^{®} ITX (isopropyl thioxanthone), SpeedCure^{®} DETX (2,4-diethylthioxanthone) or SpeedCure^{®} CPTX (1-chloro-4-propoxythioxanthone); an α-hydroxy acetophenone; an acylphosphine oxide such as SpeedCure^{®} BPO (phenyl bis(2,4,6-trimethylbenzoyl)-phosphine oxide), SpeedCure^{®} TPO (2,4,6-trimethylbenzoyldiphenylphosphine oxide) or SpeedCure^{®} TPO-L (ethyl (2,4,6-trimethylbenzoyl)phenyl phosphinate); a phenylglyoxylate such as SpeedCure^{®} MBF (methylbenzoylformate); and mixtures thereof.

The radical initiator may be present in the curable composition in an amount of from 0.1 to 5% of the total weight of polymerizable compounds in the composition.

### Ethylenically unsaturated compounds

In an embodiment, the composition may comprise one or more ethylenically unsaturated compounds other than a (meth)acrylate-functionalized monomer or oligomer. Examples of such ethylenically unsaturated compounds include:
- polyvinylic and/or polyallylic monomers (in particular divinyl benzene, 1 ,4-butanediol divinyl ether, tri(ethylene glycol) divinyl ether, diallyl ether, glycerol diallyl ether, glycerol triallyl ether, trimethylolpropane diallyl ether, trimethylolpropane triallyl ether, pentaerythritol triallyl ether, pentaerythritol tetraallyl ether, diallyl phthalate, triallyl isocyanurate, 2,4,6-triallyloxy-1,3,5-triazine, glyoxal bis(diallyl acetal) and mixtures thereof);
- vinyl esters of carboxylic acids (in particular vinyl acetate, vinyl propionate, vinyl hexanoate, vinyl 2-ethylhexanoate, vinyl octanoate, vinyl pelargonate, vinyl laurate, vinyl stearate, a vinyl ester of versatic acid and mixtures thereof);
- vinyl ethers (in particular vinyl methyl ether, vinyl ethyl ether, vinyl n-butyl ether, vinyl iso-butyl ether and mixtures thereof, ethylene glycol divinyl ether, triethylene glycol divinyl ether and trimethylolpropane trivinyl ether);
- cycloaliphatic vinyl monomers (in particular vinylcyclohexane);
- olefins (in particular ethylene, propene, 1-butene, isobutylene, diisobutylene, 1-nonene, 1-decene and mixtures thereof);
- conjugated dienes (in particular butadiene, isoprene, pentadiene, chlorodiene and mixtures thereof);
- vinyl aromatic monomers (in particular styrene, alpha-methylstyrene, tert-butylstyrene, ortho-, meta-, and para-methylstyrene, ortho-, meta- and para-ethylstyrene, o-methyl-p-isopropylstyrene, p-chlorostyrene, p-bromostyrene, o,p-dichlorostyrene, o,p-dibromostyrene, ortho-, meta- and para-methoxystyrene, optionally substituted indenes, optionally substituted vinyl naphthalenes, acenaphthylene, diphenylethylene, vinyl anthracene and mixtures thereof);
- mono- or dicarboxylic acid monomers, cyclic anhydride monomers and salts thereof (in particular 3-butenoic acid, crotonic acid, vinyl acetic acid, fumaric acid, maleic acid, maleic anhydride, tetrahydrophthalic acid, tetrahydrophthalic anhydride, itaconic acid, mesaconic acid, citraconic acid, glutaconic acid, muconic acid and mixtures thereof);
- unsaturated polymers such as polybutadiene;
- as well as the alkoxylated (e.g., ethoxylated and/or propoxylated) derivatives thereof
- and mixtures thereof.

### Amine synergist

The curable composition of the present invention may comprise an amine synergist. The curable composition may comprise a mixture of amine synergists.

Amine synergists may be introduced in the curable composition of the present invention in order to act synergistically with Norrish Type II photoinitiators and/or reduce oxygen inhibition. Amine synergists are typically tertiary amines. When used in conjunction with Norrish Type II photoinitiators, the tertiary amine provides an active hydrogen donor site for the excited triple state of the photoinitiator, thus producing a reactive alkyl-amino radical which can subsequently initiate polymerization. Tertiary amines are able to convert unreactive peroxy species, formed by reaction between oxygen and free radicals, to reactive alkyl-amino radicals, thus reducing the effects of oxygen on curing.

Examples of suitable amine synergists include low-molecular weight tertiary amines (i.e. having a molecular weight of less than 200 g/mol) such as triethanolamine and N-methyldiethanolamine. Other types of amine synergists are aminobenzoates, polymerizable aminobenzoates, polymeric aminobenzoates and mixtures thereof. Examples of aminobenzoates include ethyl 4-(dimethylamino)benzoate (EDB), pentyl 4-(dimethylamino)benzoate, 2-ethylhexyl 4-(dimethylamino)benzoate and 2-butoxyethyl 4-(dimethylamino)benzoate (BEDB).

The concentration of amine synergist in the curable composition will vary depending on the type of compound that is used. Typically, however, the curable composition is formulated to comprise from 0% to 25%, in particular 0.5% to 20%, more particularly 1 to 15%, by weight of amine synergist based on the total weight of the curable composition.

### Additives

The curable composition of the present invention may further comprise an additive. The curable composition may comprise a mixture of additives.

In particular, the additive may be selected from stabilizers, antioxidants, UV absorbers, optical brighteners, polymerization inhibitors, foam inhibitors, flow or leveling agents, colorants, pigments, dispersants (wetting agents, surfactants), slip additives, fillers, thixotropic agents, matting agents, impact modifiers, waxes and mixtures thereof; and any other additive conventionally used in the coating, sealant, adhesive, molding, 3D printing, dentistry or ink arts.

According to some embodiments, the curable composition may comprise a stabilizer.

Stabilizers may be introduced in the curable composition of the present invention in order to provide adequate storage stability and shelf life. Further, stabilizers may be used during the preparation of the curable composition, to protect against unwanted reactions during processing of the polymerizable components of the curable composition. A stabilizer may be a compound or substance which retards or prevents reaction or curing of actinically-polymerizable functional groups present in a composition in the absence of actinic radiation. However, it will be advantageous to select an amount and type of stabilizer such that the composition remains capable of being cured when exposed to actinic radiation (that is, the stabilizer does not prevent radiation curing of the composition). The stabilizer may, in particular be a free radical stabilizer (i.e. a stabilizer which functions by inhibiting free radical reactions).

Any of the stabilizers known in the art related to (meth)acrylate-functionalized compounds may be utilized in the present invention. Quinones represent a particularly preferred type of stabilizer which can be employed in the context of the present invention. As used herein, the term "*quinone*" includes both quinones and hydroquinones as well as ethers thereof such as monoalkyl, monoaryl, monoaralkyl and bis(hydroxyalkyl) ethers of hydroquinones. Hydroquinone monomethyl ether is an example of a suitable stabilizer which can be utilized. Other stabilizers known in the art are hydroquinone (HQ), 4-methoxyphenol (MEHQ), 4-tert-butylcatechol (TBC), 3,5-di-tertiobutyl-4-hydroxytoluene (BHT), phenothiazine (PTZ), pyrogallol, phosphite compounds, triphenyl antimony and tin(ll) salts.

The concentration of stabilizer in the curable composition will vary depending upon the particular stabilizer or combination of stabilizers selected for use and also on the degree of stabilization desired and the susceptibility of components in the curable compositions towards degradation in the absence of stabilizer. Typically, however, the curable composition is formulated to comprise from 5 to 5000 ppm stabilizer relative to the polymerizable compounds. According to certain embodiments of the invention, the reaction mixture during each stage of the method employed to make the curable composition contains at least some stabilizer, e.g., at least 10 ppm stabilizer relative to the polymerizable compounds.

The curable composition may comprise a colorant. A colorant may be a dye, a pigment and mixtures thereof. The term "dye", as used herein means a colorant having a solubility of 10 mg/L or more in the medium in which it is introduced at 25°C. The term "pigment" is defined in DIN 55943, as a colorant that is practically insoluble in the application medium under the pertaining ambient conditions, hence having a solubility of less than 10 mg/L therein at 25°C. The term "C.I." is used as an abbreviation for Colour Index.

The colorant may be a pigment. Organic and/or inorganic pigments may be used. The pigment may be black, cyan, magenta, yellow, red, orange, violet, blue, green, brown and mixtures thereof. Pigments may be chosen from those disclosed by HERBST, Willy, et al. Industrial Organic Pigments, Production, Properties, Applications. 3rd edition. Wiley - VCH, 2004. ISBN 3527305769.

In addition, the curable composition of this invention may comprise at least one compound selected from UV absorbers, optical brighteners and mixtures thereof. These additives are especially useful when the composition is to be used in a stereolithography process, since they allow reducing the penetration of light and thus the polymerization depth during light-induced curing, thus improving the precision of the stereolithographoic process.

Some UV absorbers also act as colorants. Examples thereof are dyes such as azo dyes, carbonyl dyes, cyanine dyes, azomethines, methines, phthalocyanines and dioxazines, preferably azo dyes; organic pigments based on benzotriazoles, benzophenones or triazines, preferably bumetrizole; and mixtures thereof. UV absorbers that may be used in this invention preferably have an absorption maximum corresponding to the wavelength of the curing light, for instance between 350 and 550 nm.

Examples of optical brighteners are compounds comprising a benzoxazolyl thiophene moiety andr terephthalic acid derivatives. Optical brighteners that may be used in this invention preferably have an absorption maximum below 400 nm.

The curable composition may comprise one or more fillers, in particular one or more inorganic fillers. As used herein, a filler is an inorganic and/or organic particle which may be used to adjust the viscosity of the curable composition as well as the mechanical and/or optical properties of the cured material. The filler or fillers preferably has/have a D50 particle size of less than 25 µm, preferably less than 10 µm and particularly preferably less than 5 µm. In a preferred embodiment, the curable composition contains a mixture of two or more fillers having different particle sizes. Such mixtures advantageously prevent an undesirable increase of viscosity of the compositions thus facilitating their use in 3D printing methods. Examples of inorganic fillers include marble, granite, quartz, diatomaceous earth, feldspar, mica, gypsum, glass, rock flour, limestone, ceramic, clay, sand, silica, alumina, titanium dioxide, magnesium oxide, zirconia, talc, aluminum hydroxide, magnesium hydroxide, calcium hydroxide, zirconium hydroxide, calcium phosphate, calcium carbonate, barium sulfate, hydroxyapatite, zeolite, and mixtures thereof. Examples of organic fillers include waxes, in particular carnauba wax, and polymer particles such as polymethyl methacrylate (PMMA) particles. The surface of the fillers can be modified in order to enhance the compatibility/dispersibility of the fillers in the curable composition. Surface-modification may be carried out by chemically bonding compounds bearing functional groups (namely acid groups, silyl groups and/or (meth)acrylate groups) to the surface of the fillers.

The curable composition of the invention may comprise a dispersant. The dispersant may be used to disperse an insoluble material such as a pigment or filler in the curable composition.

The dispersant may be a polymeric dispersant, a surfactant and mixtures thereof.

The polymeric dispersant may have a number average molecular weight Mn between 500 and 30,000 g/mol, more preferably between 1,500 and 10,000 g/mol.

### Solvents

The curable composition of the invention may comprise a solvent. As used herein, the term "solvent" means a non-reactive organic solvent, i.e. a solvent comprising carbon and hydrogen atom that does not react when exposed to the actinic radiation used to cure the curable compositions described herein.

Advantageously, the curable composition of the present invention may be formulated to be solvent-free. For example, the curable composition of the present invention may contain little or no solvent, e.g., less than 10 %, or less than 5 %, or less than 1 %, or even 0 % by weight of solvent, based on the total weight of the curable composition.

According to some embodiments, the curable composition is liquid at 25°C. In various embodiments of the invention, the curable compositions described herein are formulated to have a viscosity of less than 10,000 mPa.s, or less than 5,000 mPa.s, as measured at 25°C using a Brookfield viscometer, model DV-II, using a 27 spindle (with the spindle speed varying typically between 20 and 200 rpm, depending on viscosity). In some embodiments of the invention, the viscosity of the curable composition is from 100 to 10,000 mPa.s, for instance from 1,000 to 5,000 mPa.s. In other embodiments, the viscosity of the curable composition is from 10 to 500 mPa.s, or from 10 to 500 mPa.s, or from 10 to 250 mPa.s, or from 10 to 100 mPa.s at 25°C.

In a preferred embodiment, the curable composition of this invention is substantially free of or free of bisphenol A ("BPA") and (meth)acrylated derivatives thereof, such as bispenol A di(meth)acrylate, alkoxylated bispenol A di(meth)acrylates, bisphenol A glycidyl di(meth)acrylates. Such compositions are sometimes referred to as "BPA non-intent" because BPA, including derivatives or residues thereof, are not intentionally added but may be present in trace amounts because of unavoidable contamination from the environment. The term "substantially free of' as used in this context means the compositions contain less than 10 parts per million (ppm), whereas "free of" means less than 20 parts per billion (ppb) of any of the above-mentioned compounds, based on the total weight of the curable composition.

### Uses

The curable composition of the invention may be an ink composition, a coating composition, an adhesive composition, a sealant composition, a molding composition, a dental composition, a composite material composition, or a 3D-printing composition.

The composition according to the invention may be used to obtain a cured product.

In particular, the cured product may be an ink, a coating, an adhesive, a sealant, a molded article, a dental material, a composite material, or a 3D-printed article.

The present invention thus also relates to the use of the curable composition of the present invention as an ink composition, a coating composition, an adhesive composition, a sealant composition, a molding composition, a dental composition, a composite material composition, or a 3D-printing composition.

The present invention also relates to a method of using the curable composition described herein as an ink composition, a coating composition, an adhesive composition, a sealant composition, a molding composition, a dental composition, a composite material composition, or a 3D-printing composition.

### Cured product and method of making

The present invention also relates to a process for the preparation of a cured product. The process for the preparation of a cured product according to the invention comprises curing the curable composition of the invention. Thus, the cured product may be deemed as the reaction product of the curable composition, formed by curing.

In particular, the curable composition may be cured by exposing the composition to radiation and/or heat. More particularly, the curable composition may be cured by exposing the composition to UV, near-UV, visible, infrared, near-infrared and/or electron beam radiation. The curable composition may advantageously be cured by exposing the composition to a LED light source.

Curing may be accelerated or facilitated by supplying energy to the curable composition, such as by heating the curable composition. A curable composition may be partially cured by exposure to actinic radiation, with further curing being achieved by heating the partially cured article. For example, a product formed from the curable composition may be heated at a temperature of from 40°C to 120°C for a period of time from 5 minutes to 12 hours. Prior to curing, the curable composition may be applied to a substrate surface in any known conventional manner, for example, by spraying, jetting, knife coating, roller coating, casting, drum coating, dipping, and the like and combinations thereof. Indirect application using a transfer process may also be used.

The substrate on which the curable composition is applied and cured may be any kind of substrate. Suitable substrates are detailed below. When used as an adhesive, the curable composition may be placed between two substrates and then cured, the cured composition thereby bonding the substrates together to provide an adhered article. Curable compositions in accordance with the present invention may also be formed or cured in a bulk manner (e.g., the curable composition may be cast into a suitable mold and then cured).

The substrate may be a ceramic, metallic, mineral, cellulosic, animal-based or polymeric substrate.

The substrate may be porous or substantially non-porous. The substrate may be transparent, translucent, or opaque.

Examples of ceramic substrates include alumina-based ceramics and zirconia-based ceramics.

Examples of metallic substrates include titanium, gold, silver, copper, brass, steel and bronze.

Examples of mineral substates include glass, asbestos and basalt.

Examples of cellulosic substrates include plain paper or resin coated paper (e.g. polyethylene or polypropylene coated paper). There is no real limitation on the type of paper and it includes newsprint paper, magazine paper, office paper, wallpaper but also paper of higher grammage, usually referred to as boards, such as white lined chipboard, corrugated board and packaging board. Further examples of cellulosic substrates include bamboo, cotton, flax, hemp, jute, lyocell, modal, rayon, raffia, ramie and sisal.

Examples of cellulosic substrates include wool, fur, silk and leather.

Examples of polymeric substrates include polyethylene, polypropylene, polycarbonate, polyvinyl chloride, polyethylene terephthalate, polyethylene naphthalate, polylactide, polyamide, polyimide, polyacrylonitrile, polyurethane, acrylonitrile butadiene styrene. There is no restriction on the shape of the substrate. It can be a sheet, a film, a non-woven or woven fiber mat or a three dimensional object.

In particular, the substrate may be selected from a food and beverage packaging, a pharmaceutical packaging, a textile, a tooth, a medical device, a food and beverage processing equipment, a water pipe.

The present invention also relates to cured products comprising the cured composition of the present invention.

The cured product obtained with the process of the invention may be an ink, a coating, an adhesive, a sealant, a molded article, a dental material, a composite material or a 3D-printed article.

Preferably, the cured product is a dental material selected from a dental restoration, a prosthesis such as a denture base, a prosthesis material, an artificial tooth, an inlay, an onlay, a crown, a bridge, a drilling template, a try-in or an orthodontic appliance selected from the group consisting of aligners, positioners, aligner attachments, positioners bearing aligner attachments, occlusal splints, transfer splints and orthodontic splints.

In a preferred embodiment, at least part of the cured product is obtained with a 3D-printing process as described below.

### Process of 3D printing

The curable compositions as described herein may also be 3D-printing compositions and may be used in a 3D printing process. Accordingly, the cured product of the ivnetion mat be obtained with a 3D printing process.

Three-dimensional (3D) printing (also referred to as additive manufacturing) is a process in which a 3D article is manufactured by accretion of construction material. The 3D printed article is created by utilizing the computer-aided design (CAD) data of an object through sequential construction of two dimensional (2D) layers or slices that correspond to cross-sections of 3D objects. Such 3D printed articles may be free-standing/self-supporting and may comprise, consist essentially of, or consist of an at least partially cured composition as described herein. The three-dimensional article may also be a composite, comprising at least one component consisting essentially of or consisting of at least partially cured composition as described herein as well as at least one additional component comprised of one or more materials other than such a cured composition (for example, a metal component, a thermoplastic component, an inorganic filler and/or a fibrous reinforcement).

The 3D printed article may be obtained with a process for the preparation of a 3D printed article that comprises printing a 3D printed article with the curable compositions as described herein. In particular, the process may comprise printing a 3D printed article layer by layer or continuously.

The process for the preparation of a 3D printed article may comprise curing the curable compositions of the invention. In particular, the curable compositions may be cured by exposing the curable compositions to actinic radiation. Specifically, the actinic radiation may include UV, near-UV, visible, infrared, near-infrared and/or electron beam radiation.

A plurality of layers of a curable composition as described herein may be applied to a substrate surface; the plurality of layers may be simultaneously cured (by exposure to a single dose of radiation, for example) or each layer may be successively cured before application of an additional layer of the curable composition. In some embodiments, each layer may be successively cured before the application of an additional layer, then the plurality of layers may be simultaneously cured.

The curable compositions may be partially cured during the 3D printing process to form a green composition. As described below, the green composition may then be post-cured to form a post-cured composition. It should be understood that the cured composition may include both the green composition and the post-cured composition. Accordingly, the curable compositions may be used to construct a 3D printed article which may comprise the curable compositions, the green composition, the post-cured compositions, or a combination of these.

In particular, the process for the preparation of a 3D printed article may comprise the steps of:
a) providing (e.g., coating) a first layer of the curable composition as described herein onto a surface;
b) curing the first layer, at least partially, to provide a cured first layer;
c) providing (e.g., coating) a second layer of the curable composition as described herein onto the cured first layer;
d) curing the second layer, at least partially, to provide a cured second layer adhered to the cured first layer; and
e) repeating steps c) and d) a desired number of times to build up the 3D printed article.

The process may further comprise step f) exposing the 3D printed article to actinic radiation, wherein the actinic radiation is of sufficient intensity and duration to cause at least 50 wt. %, at least 60 wt. %, at least 70 wt. %, at least 80 wt. %, at least 90 wt. %, at least 95 wt. %, or even at least 99 wt. % of the polymerizable components in the curable composition to cure, based on the total weight of polymerizable components in the curable composition. The entirety of, or portions of, the 3D printed article may be exposed to the actinic radiation.

Although the curing steps may be carried out by any suitable means, which will in some cases be dependent upon the components present in the curable composition, in certain embodiments of the disclosure the curing is accomplished by exposing the layer to be cured to an effective amount of radiation, in particular actinic radiation as described above.

After the 3D printed article has been printed, it may be subjected to one or more post-processing steps. The post-processing steps can be selected from one or more of the following steps removal of any printed support structures, washing with water and/or organic solvents to remove residual resins, and post-curing using thermal treatment and/or actinic radiation either simultaneously or sequentially. The post-processing steps may be used to transform the freshly printed article into a finished, functional article ready to be used in its intended application.

In some embodiments of the one or more post-processing steps, curing may be accelerated or facilitated by supplying energy to the curable composition, such as by heating the curable composition. An actinically-curable composition may be cured by exposure to actinic radiation, with further curing being achieved by heating the partially cured article. For example, an article formed from the curable composition (e.g., a 3D printed article) may be heated at a temperature of from 40°C to 250°C for a period of time of from 5 minutes to 12 hours.

Suitable 3D printing processes include digital light printing (DLP), stereolithography (SLA), inkjet, multi-jet printing, piezoelectric printing, actinically-cured extrusion, liquid crystal display (LCD) printing, gel deposition printing, continuous liquid interface (or interphase) printing (CLIP), high viscosity printing using a carrier film, and combinations of any of these.

### Process of inkjet printing

The process of inkjet printing comprised jetting the curable composition of the invention onto a substrate.

The substrate on which the curable composition is jetted may be any kind of substrate. Suitable substrates are as detailed above.

The curable composition may be jetted by one or more print heads ejecting small droplets in a controlled manner through nozzles onto a substrate moving relative to the print head(s). The print head may be a piezoelectric head or a continuous type print head.

The inkjet printing process may be carried out in a single pass or with a multi-pass printing mode.

The inkjet printing process may further comprise a UV-curing step. In inkjet printing, the UV curing device may be arranged in combination with the print head of the inkjet printer, travelling therewith so that the liquid UV curable inkjet ink is exposed to curing radiation very shortly after been jetted.

In a particularly preferred embodiment, the UV curing step is performed using UV LED light sources. According to certain embodiments of the invention, curing may be performed by exposing the curable composition to light having a wavelength of 350 nm to 490 nm, or 365 nm to 465 nm, or 380 nm to 410 nm. The light intensity may be, for example, from 20 mW/cm² to 150 mW/cm², or 40 mW/cm² to 90 mW/cm². The curable composition may be stationary when exposed to light. Alternatively, the curable composition may be in motion when exposed to light (for example, on a conveyor belt).

For facilitating curing, the inkjet printer may include one or more oxygen depletion units. The oxygen depletion units place a blanket of nitrogen or other relatively inert gas (e.g. CO₂), with adjustable position and adjustable inert gas concentration, in order to reduce the oxygen concentration in the curing environment.

The cured product obtained according to one of the above processes may have a reduced amount of extractables.

The reduction in the amount of extractables may be assessed in comparison with a cured product obtained with a conventional photoinitiator.

The extractables may be any component that migrates from the cured product. In particular, the extractables may be a photoinitiator or a residue thereof.

Migration in inkjet inks may occur in different ways:
- Penetration Migration - through the substrate to the reverse-side of the print;
- Set-off Migration - from the printed side of a substrate to the reverse side of the substrate while stacked or stored on a roll;
- Vapor-phase migration - evaporation of volatile compounds when heated;
- Condensation Extraction - condensation of critical compounds when cooked or sterilized.

The amount of extractables may be determined quantitatively using a suitable analytical method such as liquid chromatography mass spectroscopy (LC-MS). For example, the curable composition can be applied in 12 µm thickness film on a glass substrate, and crosslinked using a UV Hg lamp. Resulting cured films are removed from the glass plate, weighed and soaked in solvent such as acetonitrile or dichloromethane. The liquid fraction is finally evaporated and the residue, corresponding to the extractables part, is weighed, allowing to determine the amount of product that is uncured (not trapped within the photocured network).

Analytical methods such as nuclear magnetic resonance (NMR), liquid chromatography mass spectroscopy (LC-MS) or gas chromatography mass spectroscopy (GC-MS) may then be used to identify the nature of the extractables and refine their corresponding content.

In particular, the cured product may have less than 5 %, less than 2 %, less than 1 %, less than 0.5 %, less than 0.25 % or less than 0.1 %, by weight of extractables based on the weight of the cured product.

Although the invention is illustrated and described herein with reference to specific embodiments, the invention is not intended to be limited to the details shown. Rather, various modifications may be made in the details within the scope and range of equivalents of the claims and without departing from the invention.

### EXAMPLES

The following examples illustrate the invention without limiting it.

### Example 1: Synthesis of ethoxylated isosorbide dimethacrylate

In a three round-bottom flask equipped with an air sparge, a condenser and a Dean-stark device, propoxylated isosorbide (1,5PO) (1.72 mole, 1 mol. equivalent) is stirred at room temperature with methacrylic acid (MAA) (4.16 mole, 1.2 mol. equivalent), hydroquinone (5,300 ppm), phenothiazine (18 ppm) and a 50:50 wt. mixture of toluene and heptane (20% wt. of the total batch size).

The catalyst, methyl sulfonic acid (MSA) (0,3 mole) is then introduced, and the temperature is increased until reflux is obtained, around 115°C. The esterification proceeds until the conversion of MAA reaches at least 94%.

At the end of the esterification, the catalyst and the residual methacrylic acid are neutralized with an aqueous solution of NaOH 25% wt., at 40°C for 2 min. The aqueous NaOH 25% amount is calculated to be 1.05 molar equivalent regarding the total acid quantity remaining in the reaction medium (coming from both MSA and MAA).

The organic and aqueous phases are then segregated, and the organic phase undergoes three more liquid-liquid extractions with NaOH 25% wt., at 45°C for 15 min. For each of these washings, the aqueous NaOH 25% quantity is calculated to be 1.8 times by weight the amount of the organic phase. A final liquid-liquid extraction is then performed by using an aqueous solution of NaCl 11% wt., at 50°C for 5 min. The aqueous NaCl 11% quantity is calculated to be 2 times by weight the amount of the organic phase.

Once the organic phase is recovered, the solvent is finally stripped out from the organic phase under reduced pressure and propoxylated (1.5 PO) isosorbide dimethacrylate is obtained with a yield of 91 %.

### Example 2: Preparation of curable compositions

Curable compositions were prepared by mixing the constituents listed in Table 1 at 60°C with an overhead mixer, in the proportions given below (in % by weight based on the total weight of the formulation, except otherwise provided), wherein the oligomers were preheated to 60°C.

**Table 1**

| Constituent | Ex 2-1 (inv) | Ex 2-2 (comp) | Ex 2-3 (comp) | Ex 2-4 (inv) | Ex 2-5 (comp) | Ex 2-6 (comp) |
|---|---|---|---|---|---|---|
| Difunctional aliphatic urethane methacrylate oligomer (CN1970EU from Arkema) | 45% | 45% | 45% | - | - | - |
| Difunctional aliphatic urethane acrylate oligomer (CN991 from Arkema) | - | - | - | 15% | 15% | 15% |
| Triethylene glycol dimethacrylate (SR205 from Arkema) | - | - | - | 20% | 20% | 20% |
| Tricyclodecanedimethanol Acrylate (SR789 from Arkema) | 30% | 30% | 30% | 20% | 20% | 20% |
| Propoxylated (1.5 PO) isosorbide dimethacrylate of Example 1 | 25% | - | - | 25% | - | - |
| Ethoxylated (3 EO) Bisphenol A dimethacrylate (SR348C from Arkema) | - | 25% | - | - | 25% | - |
| Ethoxylated (4 EO) Bisphenol A dimethacrylate (SR540 from Arkema) | - | - | 25% | - | - | 25% |
| 2,4,6-trimethylbenzoyl diphenylphosphine oxide (Speedcure^{®} TPO-L from Arkema) | 1 phr* | 1 phr* | 1 phr* | 1 phr* | 1 phr* | 1 phr* |

| | | | | | | |
|---|---|---|---|---|---|---|
| * parts by weight relative to 100 parts by weight of the polymerizable compounds. | | | | | | |

### Example 3: Curing Experiments

The following example illustrates the curing properties of the compositions of Example 2. Samples of the curable compositions of Example 2 were cured in silicone molds under LED (395 nm). The exposure time was controlled by a conveyor belt that passed the mold underneath the LED at approximately 10 m/min. Ten passes were done for each sample The samples were then post-cured for 2 hours at 90°C in an oven.

The mechanical properties of the cured samples were then assessed as follows.

### Tensile testing (tensile stress, tensile modulus and elongation)

Elongation at break (machine direction), tensile stress and modulus were measured according to the ISO 527/2 standard with an Instron 68TM5 testing device on cured samples using the following parameters:
Speed: 1^{st} (from 0,05% to 0,25%) = 0,25 mm/min; 2^{nd} = 5 mm/min
Sample: Type 5A (long tab)
Extensometer Clip-on

### Shore D hardness:

This property was measured using the NF ISO 868 standard with a Zwick Durometer. The value obtained after 15 seconds contact between the indenter and the sample was recorded.

In addition, the viscosity of the curable compositions of Example 2 was measured at 25°C using a rotational Brookfield viscometer DV2T LV fitted with an S34 mobile for 2,000 mPa.s viscosity or S27 mobile for lower than 1,000 mPa.s.

The results are detailed in Table 2 below.

**Table 2**

| | Ex 2-1 (inv) | Ex 2-2 (comp) | Ex 2-3 (comp) | Ex 2-4 (inv) | Ex 2-5 (comp) | Ex 2-6 (comp) |
|---|---|---|---|---|---|---|
| Viscosity @25°C [mPa.s] | 1379 | 2160 | 2030 | 65 | 121 | 123 |
| Shore D hardness [°SH] | 80 | 78 | 77 | 79 | 76 | 76 |

| Tensile strength : | | | | | | |
|---|---|---|---|---|---|---|
| Stress @ break [MPa] | 67 | 52 | 49 | 67 | 61 | 57 |
| Elongation @ break [%] | 5 | 6 | 5 | 5 | 5 | 5 |
| Young modulus [GPa] | 3,2 | 2,8 | 2,6 | 3,2 | 2.8 | 2.8 |

As can be seen from these results, the samples obtained from the curable composition of this invention (Ex 2-1, respectively Ex 2-4) have mechanical properties similar, or even increased (in particular stress at break and Young Modulus), as well as a reduced viscosity, compared to the samples obtained from the comparative compositions (Ex 2-2 and Ex 2-3, respectively Ex 2-5 and 2-6). They can thus be used to prepare dental parts free of BPA (in particular dental models, try-ins, denture teeth, denture base, Crown and bridges and orthodontic appliances) by additive manufacturing methods such as stereolithography.

### Comparative Example 4: Preparation of curable compositions

Curable compositions were prepared by mixing the constituents listed in Table 3 at 60°C with an overhead mixer, in the proportions given below (in % by weight based on the total weight of the formulation, except otherwise provided), wherein the oligomers were preheated to 60°C, when present.

**Table 3**

| Constituent | Ex 2-1 (inv) | Ex 3-1 (comp) | Ex 3-2 (comp) |
|---|---|---|---|
| Difunctional aliphatic urethane methacrylate oligomer (CN1970EU from Arkema) | 45% | 64.3% | - |
| Difunctional aliphatic urethane acrylate oligomer (CN991 from Arkema) | - | - | - |
| Triethylene glycol dimethacrylate (SR205 from Arkema) | - | - | - |
| Tricyclodecanedi methanol Acrylate (SR789 from Arkema) | 30% | - | 54.5% |
| Propoxylated (1.5 PO) isosorbide dimethacrylate of Example 1 | 25% | 35.7% | 45.5% |
| 2,4,6-trimethylbenzoyl diphenylphosphine oxide (Speedcure^{®} TPO-L from Arkema) | 1 phr* | 1 phr* | 1 phr* |

| | | | |
|---|---|---|---|
| * parts by weight relative to 100 parts by weight of the polymerizable compounds. | | | |

### Comparative Example 5: Curing experiments

The following example illustrates the curing properties of the compositions of Comparative Example 4.

Samples of the curable compositions of Comparative Example 4 were cured in silicone molds under LED (395 nm). The exposure time was controlled by a conveyor belt that passed the mold underneath the LED at approximately 10 m/min. Eight passes were done for each sample The samples were then post-cured for 2 hours at 90°C in an oven.

The mechanical properties and the viscosity of the cured samples were then assessed as explained in Example 3.

The results are detailed in Table 4 below.

**Table 4**

| | Ex 2-1 (inv) | Ex 3-1 (comp) | Ex 3-2 (comp) |
|---|---|---|---|
| Viscosity @25°C [mPa.s] | 1379 | 23940 | 33 |
| Shore D hardness [°SH] | 80 | 81 | 81 |

| Tensile strength : | | | |
|---|---|---|---|
| Stress @ break [MPa] | 67 | 85 | 57 |
| Elongation @ break [%] | 5 | 4 | 2 |
| Young modulus [GPa] | 3,2 | 3,4 | 4,2 |

As can be seen from these results, the sample obtained from the curable composition of this invention (Ex 2-1) has a reduced viscosity, compared to the sample obtained from the composition deprived of (meth)acrylate-functionalized monomer (Ex 3-1). In addition, it has improved mechanical properties compared to the composition deprived of (meth)acrylate-functionalized oligomer (Ex 3-2).

## Claims

1. A curable composition comprising:
a) at least one alkoxylated isosorbide di(meth)acrylate,
b) at least one (meth)acrylate-functionalized oligomer, and
c) at least one (meth)acrylate-functionalized monomer.

2. The curable composition according to claim 1, **characterized in that** component c) comprises or consists of at least one (meth)acrylate-functionalized monomer selected from mono-(meth)acrylate esters of aliphatic alcohols; mono-(meth)acrylate esters of aromatic alcohols; mono-(meth)acrylate esters of oligomeric and polymeric glycols such as diethylene glycol, triethylene glycol, dipropylene glycol, tripropylene glycol, polyethylene glycol, and polypropylene glycol; mono-(meth)acrylate esters of monoalkyl ethers of glycols and oligoglycols; mono-(meth)acrylate esters of alkoxylated aliphatic alcohols; mono-(meth)acrylate esters of alkoxylated aromatic alcohols; caprolactone mono(meth)acrylates; and mixtures thereof.

3. The curable composition according to claim 1 or 2, **characterized in that** component c) comprises or consists of at least one (meth)acrylate-functionalized monomer selected from poly(meth)acrylate ester of polyols, such as ethylene glycol di(meth)acrylate; diethylene glycol di(meth)acrylate; triethylene glycol di(meth)acrylate; tetraethylene glycol di(meth)acrylate; polyethylene glycol di(meth)acrylate; propylene glycol di(meth)acrylate; dipropylene glycol di(meth)acrylate; tripropylene glycol di(meth)acrylate; tetrapropylene glycol di(meth)acrylate; polypropylene glycol di(meth)acrylate; polytetramethylene glycol di(meth)acrylate; 1,2-butanediol di(meth)acrylate; 2,3-butanediol di(meth)acrylate; 1,3-butanediol di(meth)acrylate; 1,4-butanediol di(meth)acrylate; 1,5-pentanediol di(meth)acrylate; 1,6-hexanediol di(meth)acrylate; 1,8-octanediol di(meth)acrylate; 1,9-nonanediol di(meth)acrylate; 1,10-nonanediol di(meth)acrylate; 1,12-dodecanediol di(meth)acrylate; neopentyl glycol di(meth)acrylate; 2-methyl-2,4-pentanediol di(meth)acrylate; polybutadiene di(meth)acrylate; cyclohexane-1,4-dimethanol di(meth)acrylate; tricyclodecane dimethanol di(meth)acrylate;; glyceryl di(meth)acrylate; glyceryl tri(meth)acrylate; trimethylolethane tri(meth)acrylate; trimethylolethane di(meth)acrylate; trimethylolpropane tri(meth)acrylate; trimethylolpropane di(meth)acrylate; pentaerythritol di(meth)acrylate; pentaerythritol tri(meth)acrylate; pentaerythritol tetra(meth)acrylate, di(trimethylolpropane) diacrylate; di(trimethylolpropane) triacrylate; di(trimethylolpropane) tetraacrylate, sorbitol penta(meth)acrylate; di(pentaerythritol) tetraacrylate; di(pentaerythritol) pentaacrylate; di(pentaerythritol) hexa(meth)acrylate; tris (2-hydroxyethyl) isocyanurate tri(meth)acrylate; and mixtures thereof.

4. The curable composition according to any one of claims 1 to 3, **characterized in that** component c) represents from 5 to 70 wt.%, preferably from 10 to 60 wt.%, more preferably from 20 to 50 wt.% of the total weight of polymerizable compounds in the composition.

5. The curable composition according to any one of claims 1 to 4, **characterized in that** component a) comprises or consists of at least one alkoxylated isosorbide di(meth)acrylate having from 1 to 5 oxyalkylene moieties selected from oxyethylene, oxypropylene, oxybutylene and mixtures thereof, preferably at least one alkoxylated isosorbide di(meth)acrylate having from 1 to 5 oxypropylene moieties; more preferably at least one alkoxylated isosorbide dimethacrylate with 1 to 5 oxypropylene moieties.

6. The curable composition according to any one of claims 1 to 5, **characterized in that** component a) represents from 5 to 70 wt.%, preferably from 10 to 60 wt.%, more preferably from 20 to 50 wt.% of the total weight of polymerizable compounds in the composition.

7. The curable composition according to any one of claims 1 to 6, **characterized in that** component b) comprises or consists of at least one (meth)acrylate-functionalized oligomer having a number average molecular weight equal or more than 600 g/mol, in particular 800 to 15,000 g/mol, more particularly 1,000 to 5,000 g/mol.

8. The curable composition according to any one of claims 1 to 7, **characterized in that** component b) comprises or consists of at least one (meth)acrylate-functionalized oligomer having 1 to 10 (meth)acrylate groups, in particular 2 to 6 (meth)acrylate groups, more particularly 2 (meth)acrylate groups.

9. The curable composition according to any one of claims 1 to 8, **characterized in that** component b) comprises or consists of at least one (meth)acrylate-functionalized oligomer selected from the group consisting of: (meth)acrylate-functionalized urethane oligomers, (meth)acrylate-functionalized epoxy oligomers, (meth)acrylate-functionalized polydiene oligomers, (meth)acrylate-functionalized polycarbonate oligomers, (meth)acrylate-functionalized polyester oligomers and mixtures thereof, preferably the (meth)acrylate-functionalized oligomer comprises or consists of a (meth)acrylate-functionalized urethane oligomer and/or a (meth)acrylate-functionalized epoxy oligomer, more preferably an aliphatic (meth)acrylate-functionalized urethane oligomer.

10. The curable composition according to any one of claims 1 to 9, **characterized in that** component b) represents from 5 to 60 wt.%, preferably from 10 to 55 wt%., more preferably from 10 to 50 wt.% of the total weight of polymerizable compounds in the composition.

11. The curable composition according to any one of claims 1 to 10, which further comprises at least one radical initiator, more preferably a radical initiator selected from: peroxides such as dialkyl, diaryl and aryl/alkyl peroxides, hydroperoxides, percarbonates, peresters, peracids, and acyl peroxides; photoinitiators such as benzoins, benzoin ethers, acetophenones, α-hydroxy acetophenones, benzil, benzil ketals, anthraquinones, phosphine oxides, acylphosphine oxides, α-hydroxyketones, phenylglyoxylates, α-aminoketones, benzophenones, thioxanthones, xanthones, acridine derivatives, phenazine derivatives, quinoxaline derivatives, triazine compounds, benzoyl formates, aromatic oximes, metallocenes, acylsilyl or acylgermanyl compounds, camphorquinones, polymeric derivatives thereo;, and mixtures thereof.

12. A process for the preparation of a cured product, comprising curing the curable composition according to any one of claims 1 to 11, preferably by exposing the curable-composition to radiation such as UV, near-UV, visible, infrared, near-infrared and/or electron beam radiation.

13. The process of claim 12 wherein the cured product is obtained with a 3D printing process.

14. The process according to claim 12 or 13, **characterized in that** the cured product is an ink, a coating, an adhesive, a sealant, a molded article, a 3D-printed article, a composite material, or a dental material.

15. The process according to claim 14, **characterized in that** the cured product is a dental material selected from a dental restoration, a prosthesis such as a denture base, a prosthesis material, an artificial tooth, an inlay, an onlay, a crown, a bridge, a drilling template, a try-in or an orthodontic appliance selected from the group consisting of aligners, positioners, aligner attachments, positioners bearing aligner attachments, occlusal splints, transfer splints and orthodontic splints.
